Europäisches Patentamt

European Patent Office

Office européen des brevets

(19)

(11) Publication number: **0 312 253 B1**

(12) **EUROPEAN PATENT SPECIFICATION**

(45) Date of publication of patent specification: **12.05.93**  (51) Int. Cl.⁵: **C07C 209/04**, C07C 211/07

(21) Application number: **88309343.7**

(22) Date of filing: **07.10.88**

Divisional application 92200338.9 filed on 07/10/88.

(54) **Process for preparing n-substituted amine.**

(30) Priority: **16.10.87 JP 261366/87**
**16.10.87 JP 261367/87**
**16.10.87 JP 261368/87**
**16.10.87 JP 261369/87**

(43) Date of publication of application:
**19.04.89 Bulletin 89/16**

(45) Publication of the grant of the patent:
**12.05.93 Bulletin 93/19**

(84) Designated Contracting States:
**DE ES**

(56) References cited:
**DE-A- 3 034 433**
**DE-A- 3 641 666**
**US-A- 4 625 063**

(73) Proprietor: **Kao Corporation**
**14-10, Nihonbashi Kayabacho 1-chome**
**Chuo-Ku Tokyo 103(JP)**

(72) Inventor: **Abe, Hiroshi**
**1450 Nishihama**
**Wakayama-shi Wakayama(JP)**
Inventor: **Aikawa, Jun**
**1130, Nishihama**
**Wakayama-shi Wakayama(JP)**
Inventor: **Okabe, Kazuhiko**
**283-28, Hatage Iwadecho**
**Naga-gun Wakayama(JP)**
Inventor: **Sotoya, Kohshiro**
**446-26, Nakaguro Iwadecho**
**Naga-gun Wakayama(JP)**

(74) Representative: **Adkins, Michael et al**
**Withers & Rogers 4 Dyer's Buildings Holborn**
**London EC1N 2JT (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

The present invention relates to a process for preparing an N−substituted amine which comprises reacting an alcohol or an aldehyde with ammonia or a primary or secondary amine.

The amine prepared according to the present invention is a material important from the industrial viewpoint as intermediates of a rust preventive, a surfactant, a germicide, a dyeing assistant and a fiber softener, etc.

It is known in the art that an alcohol or an aldehyde is reacted with ammonia or a primary or secondary amine to give the corresponding amine. However, it was difficult to selectively prepare a particular amine through a reaction of an alcohol or the like with an amine or the like.

Examples of the process for preparing an amine from an alcohol and an amine include those disclosed in Japanese Patent Laid−Open Nos. 19604/1977 (a copper chromite catalyst and a cobalt catalyst) and 59602/1978 (copper/molybdenum and copper/tungsten catalysts), U.S. Patent No. 3 223 734 (Raney nickel catalyst and copper chromite catalysts), West German Patent Laid−Open No. 1,493,781 (a supported cobalt catalyst), Japanese Patent Publication No. 55704/1982 (a copper/nickel catalyst), etc. However, these catalysts are insufficient in both the catalytic activity and the selectivity and should be used in a large amount, which brings about a low yield of the intended amine. In order to solve these problems, processes described in Japanese Patent Laid−Open Nos. 15865/1986, 149646/1987, 149647/1987, and 149648/1987 have been developed. In these processes, a copper/nickel/group VIII platinum metal element catalyst is used to prepare an intended amine in a high yield. That is, in these processes, an intended amine is prepared in a high yield through the addition of a small amount of the group VIII platinum metal element to a conventional copper/nickel catalyst having insufficient activity and selectivity for the purpose of improving the activity and selectivity.

However, the process in which the reaction is conducted in the presence of this catalyst is not always satisfactory from the viewpoint of the durability of the catalyst. Specifically, although this process is superior in the activity and the selectivity to other general processes and brings about little or no lowering in the activity of the catalyst even after recovery and reuse of tens of times, the selectivity is lowered. Therefore, with the consideration of the preparation of an N−substituted amine on a commercial scale, a further improvement in the durability is desired from the viewpoint of the yield and quality of the formed N− substituted amine.

However, N−substituted amines which have been prepared in the presence of these catalysts bring about the deterioration of hue when converted into a quaternary ammonium salt (tetraalkylammonium salt, trialkylbenzylammonium salt, etc.) which unfavourably causes remarkable deterioration of performance in applications such as surfactants.

DE−A−3641666 discloses the preparation of a secondary amine by reacting an alcohol or aldehyde in the presence of a catalyst of copper, nickel, and a platinum group metal at particular temperatures and pressures, with removal of the water formed in the reaction.

DE−A−403433 discloses a process for the preparation of a tertiary amine, in which particular alcohols, aldehydes or ketones are reacted at 150−300°C in the presence of a homogeneous two or three component colloidal catalyst containing a carboxylic acid salt.

The invention provides a process for preparing an N−substituted amine, which comprises the step of reacting an alcohol or an aldehyde with ammonia, a primary amine or a secondary amine at 150 to 250°C at a pressure from atmospheric pressure to 100 atm (1 atm = 1•013 bar) gauge, while water formed in the reaction is being removed out, in the presence of a catalyst comprising (1) copper, (2) a metal selected from the group consisting of chromium, manganese, iron and zinc and (3) a metal of a platinum VIII group.

It is preferable that the molar ratio of component (1) to (2) ranges from 10/90 to 99/1, preferably 1/9 to 9/1 or 50/50 to 99/1, the molar ratio of the component (3) to the sum total of (1) and (2) ranges from 0.001 to 0.1.

The invention will be explained in details in reference to the catalyst.

Accordingly, the present inventors have made extensive and intensive studies on an increase in the activity and selectivity through the addition of a small amount of a group VIII platinum metal element to a fourth period transition metal element catalyst (except for nickel) having insufficient activity and selectivity and, as a result, have found that the addition of a small amount of a group VIII platinum metal element to a fourth period transition metal element selected from among chromium, manganese, iron, and zinc brings about a remarkable increase in the activity and selectivity of a catalyst and enables the formation of a catalyst having activity and selectivity equal or superior to those of the copper/nickel group VIII platinum metal element catalyst. Platinum, palladium, ruthenium, and rhodium were effective as the group VIII platinum metal element.

Consequently, the present inventors have developed a high performance amination catalyst having activity and selectivity equal or superior to those of the copper/nickel/group VIII platinum metal element catalyst through the use of inexpensive and easily available other fourth period transition metal elements, i.e., chromium, manganese, iron, or zinc instead of nickel in the copper/nickel/group VIII platinum metal element catalyst.

Specifically, the present invention provides a process for preparing an N-substituted amine in a high yield which comprises reacting an alcohol or an aldehyde with ammonia or a primary or secondary amine, characterized in that the reaction is conducted in the presence of a copper/fourth period transition metal element/group VIII platinum metal element catalyst at a temperature of 150 to 250°C under atmospheric pressure or a pressure up to 100 atm (gauge pressure) while removing water formed during the reaction.

In the process of the present invention, the activity of the catalyst is so high that it is possible to conduct the reaction under mild conditions with simple equipment and to complete the reaction in a short time even when the catalyst is present in a very small amount.

The catalyst of the present invention exhibits activity several times higher than that of the copper/nickel catalyst described in Japanese Patent Publication No. 55704/1982 as well as excellent selectivity of the reaction and has performance equal or superior to that of the copper/nickel/group VIII platinum metal element catalyst. Further, the catalyst of the present invention is lower in cost and higher in availability than those of the copper/nickel/group VIII platinum metal element catalyst.

Further, the copper/fourth period transition metal element/group VIII platinum metal element catalyst of the present invention has excellent durability and has a feature that there occurs little or no lowering in the catalytic activity even after recovery and reuse of several times to tens of times.

The catalyst of the present invention exhibits activity and selectivity much higher than those of the conventional catalysts, which enables the reaction to be conducted at a low temperature under atmospheric pressure, the necessary amount of the catalyst to be decreased, and an N-substituted amine to be prepared in a high yield also from the corresponding branched aliphatic alcohol or aldehyde by virtue of an improvement in the selectivity of the reaction as opposed to the prior art in which the intended N-substituted amine could not be prepared in a high yield. Further, it is possible to prepare an N-substituted amine in a very high yield also from the corresponding polyhydric alcohol which generally causes a difficulty in increasing the yield of the product and preparing a high-quality product due to the occurrence of a side reaction.

It is necessary that the catalyst used in the present invention contain copper, a fourth period transition metal element, and a group VIII platinum metal element (hereinafter abbreviated to "platinum group element"). In the catalyst metal composition used, the proportions of copper, the fourth period transistion metal element and the platinum group element may be arbitrary. However, the molar ratio of copper to the fourth period transition metal element is 10/90 to 99/1, preferably 1/9 to 9/1, more preferably 50/50 to 99/1, and the ratio of amount of addition of the platinum group element to the total of copper and the fourth period transition metal element is preferably 0.001 to 0.1 (molar ratio), more preferably 0.001 to 0.05.

The fourth period transition metal elements particularly suitable for the reaction according to the present invention are chromium, manganese, iron, and zinc, while the platinum group elements particularly suitable for the reaction according to the present invention are platinum, palladium, ruthenium, and rhodium.

Although the catalyst metal composition should contain three components, i.e., copper, a fourth transition metal element, and a platinum group element, the catalyst suitable for the present invention may have various forms.

Specifically, in the present invention, only when the three components, i.e., copper, a fourth period transition metal element and a platinum group element, are present in the reaction system as the catalyst composition, an effect can be attained through interaction among the three components. Therefore, this three-component composition has a substantial function as a catalyst, and in the reaction the catalytic activity is not developed until each metallic component is reduced in a hydrogen atmosphere. For this reason, in the present invention, there is no limitation with respect to the form of the metals before the reduction and the state of the system after the completion of the reduction as far as the reduction in a hydrogen atmosphere according to the method described in the present specification brings about interaction among copper, the fourth period transition metal element, and the platinum group element.

Therefore, the metals suitable for the process of the present invention may have any of the following forms as far as the three metal components indispensable to the present invention bring about interaction thereamong through reduction in a hydrogen atmosphere:

    1) a form of a metal, an oxide or a hydroxide thereof and a mixture thereof which can be dispersed in a reaction medium;

2) a form of either a mixture of copper, a fourth period transition metal element and a platinum group element respectively supported on suitable carriers, or three components, i.e., copper, a fourth period transition metal element and a platinum group element, homogeneously supported on a single carrier which can be dispersed in a reaction medium;

3) a form of an aliphatic carboxylic acid salt of these metals, a complex of these metals stabilized by a suitable ligand or the like which is converted into a metallic colloid in a reaction medium to form a homogeneous system; and

4) a mixture of a form described in the above items 1) and 2) which is dispersed in a reaction medium with a form described in the above item 3) which forms a homogeneous system in a reaction system, or a form which is in a dispersed state before hydrogen reduction but becomes homogeneous after hydrogen reduction.

With respect to the form of the catalyst used in the process of the present invention, it is preferred from the viewpoint of the stabilization of the catalyst metal, i.e., immobilization of the active surface, and resistance to a catalyst poison that the above-described three metal components are homogeneously supported on a suitable carrier.

When the three metal components according to the present invention, i.e., copper, a fourth period transition metal element, and a platinum group element, are to be supported on a carrier, suitable carriers are those commonly employed as catalyst carriers, e.g., alumina, silica/alumina, diatomaceous earth, silica, active carbon, and natural and artificial zeolite. Although the amount of the catalyst metal supported on a carrier may be arbitrarily determined, it is generally preferably 5 to 70 %.

Further, these three metal components may be supported on a carrier by various methods. In this case, the form of the raw metals of the catalyst may be an oxide, a hydroxide, or various salts of copper, a fourth period transition metal element, and a platinum group element. Examples of the form of the raw metals include chlorides, sulfates, nitrates, acetates, aliphatic carboxylates of copper, a fourth period transition metal element, and a platinum group element, or complexes of these metals, e.g., acetylacetone complexes and dimethylglyoxime complexes of copper, a fourth period transition metal element, and a platinum group element. Further, with respect to the platinum group element, carbonyl complexes, amine complexes, phosphine complexes, etc. may also be employed. The preparation of a catalyst through supporting of the metal components on a carrier by making use of these raw metal materials may be conducted by any of conventional known processes, e.g., a process which comprises adding a carrier to a solution of suitable salts of copper, a fourth period transition metal element and a platinum group element to sufficiently impregnate the carrier with the solution and drying and baking the impregnated carrier (impregnation process), a process which comprises sufficiently mixing a carrier with an aqueous solution of suitable salts of copper, a fourth period transition metal element and a platinum group element and adding an aqueous alkaline solution, such as an aqueous sodium carbonate or sodium hydroxide solution or aqueous ammonia, to the mixture to precipitate the metal salts on the carrier, a process which comprises simultaneously adding an aqueous solution of suitable salts of copper, a fourth period transition metal element and a platinum group element and an aqueous alkaline solution, such as an aqueous sodium carbonate or sodium hydroxide solution or aqueous ammonia, to a water slurry of a carrier in such a manner that the pH value of the slurry remains constant (e.g., a constant pH value of 7) to precipitate the metal salts on the carrier (the above two processes are called "coprecipitation process"), a process which comprises ion exchanging sodium, potassium or the like with copper, a fourth period transition metal element and a platinum group element on zeolite (ion exchange process), and a process which comprises heat melting copper, a fourth period transition metal element, a platinum group element and an aluminum metal, cooling the resulting melt to form an alloy and eluting aluminum contained in the alloy with an alkali such as sodium hydroxide (alloy process). In the case of the coprecipitation process, the carrier is sufficiently washed with water after deposition of the metals, dried at about 100°C, and baked at 300 to 700°C to prepare a catalyst.

Another effective process comprises supporting only copper, or only copper and a fourth period transition metal element on a carrier through the above-described processes and adding a fourth period transition metal element and a platinum group element, or a supported platinum group element prior to the reaction, or an aliphatic carboxylate or a complex thereof to form a composite comprising a combination of copper with the fourth period transition metal element and the platinum group element in a reaction medium in a hydrogen atmosphere.

With respect to the form of the catalyst, it is more preferable that the three components be homogeneously supported on the same carrier.

The three components, i.e., copper, a fourth period transition metal element and a platinum group element, are indispensable to the present invention.

4

The alcohol or aldehyde which is a starting material used in the present invention is a straight – chain or branched saturated or unsaturated aliphatic alcohol or aldehyde having 8 to 36 carbon atoms. Examples of the alcohol include octyl alcohol, lauryl alcohol, myristyl alcohol, stearyl alcohol, behenyl alcohol, oleyl alcohol, and a mixture thereof, a Ziegler alcohol prepared by the Ziegler process, an oxo alcohol prepared by the oxo process, and alcohols having a branched chain such as Guerbet alcohol. Examples of the aldehyde include lauraldehyde, oxo aldehyde, and aldehydes corresponding to the above – described alcohols.

Further, various polyhydric alcohols may also be used, and examples thereof include 1,3 – butanediol, 1,4 – butanediol, 1,5 – pentanediol, 1,6 – hexanediol, 1,9 – nonanediol, diethylene glycol, and triethylene gly – col. Examples of other alcohol include aromatic alcohols, such as benzyl alcohol and phenethyl alcohol, polyoxy ether alcohols, such as adduct of an aliphatic alcohol with ethylene oxide or propylene oxide, and amino alcohols, such as ethanolamine and diethanolamine.

The alcohol or aldehyde is particularly preferably an aliphatic alcohol or aldehyde selected from among saturated and unsaturated straight – chain and branched aliphatic alcohols and aldehydes having 8 to 36 carbon atoms and aliphatic glycols having 2 to 12 carbon atoms. The amine which is reacted with these alcohols or aldehydes may be a gaseous or liquid one, and examples thereof include aliphatic amines, e.g., primary amines such as monomethylamine, ethylamine and dodecylamine, and secondary amines such as dimethylamine, diethylamine and didodecylamine.

In the present invention, it is necessary to remove water formed during a reaction of an alcohol or an aldehyde with an amine from the reaction system. When the formed water is not removed from the reaction system, no sufficient catalytic performance according to the present invention can be attained. That is, the catalytic activity and selectivity are lowered, so that it becomes difficult to easily prepare an N – substituted amine in a high yield. For example, when the reaction is conducted by making use of dimethylamine as the amine without removing the formed water, not only the amount of by – products, such as monoalkyl – methylamine, which are difficult to separate through mere distillation is increased but also a high – boiling material, such as condensate of an aldehyde, is formed in a large amount, which brings about a lowering in the yield of the intended N – substituted amine.

Water may be intermittently or continuously removed during the reaction as far as the formed water is properly removed so as not to remain in the reaction system for a long period of time. It is preferred to continuously remove water each time it is formed. More specifically, it is a common practice to introduce a suitable amount of hydrogen gas into the reaction system to distill the formed water and the excess amine (in the case of use of a gaseous amine) together with the hydrogen gas. In this method, it is also possible to reuse the hydrogen gas through condensation and separation of the formed water in a condenser. Further, a suitable solvent may be added to the reaction system, followed by distillation and removal of the formed water in the form of an azeotrope with the solvent.

In the process of the present invention, although a catalyst which has separately been reduced with a hydrogen gas may be employed, a catalyst before reduction is placed in a reactor together with the starting material of the reaction, i.e., an alcohol or an aldehyde, followed by elevation of the temperature to the reaction temperature while introducing a hydrogen gas, thereby conducting reduction. That is, the copper/fourth period transition metal element/platinum group element catalyst according to the present invention has also a significant feature that since the reduction temperature is low, the reduction can be conducted in the step of elevating the temperature to the reaction temperature.

The preferred embodiments of the process of the present invention will now be briefly described.

A reaction vessel equipped with a tube for introducing hydrogen and an amine and a condenser and a separator for condensing and separating water formed during the reaction, the excess amine and oleaginous matter distilled off is charged with a starting material, i.e., an alcohol or an aldehyde, and a catalyst. The amount of the catalyst may be arbitrary. Since, however, the catalyst of the present invention has high activity, it is usually employed in an amount as small as 0.1 to 2 % by weight based on the feed alcohol or aldehyde. The system is purged with a nitrogen gas, and the temperature is then raised while introducing hydrogen. The reaction is usually conducted at a temperature of 150 to 250˚c. However, the reaction temperature may be one outside the above – described range depending upon the kind of the reaction. The catalyst is converted into a reduced, active state during the elevation of the temperature. When the temperature reaches a predetermined value, ammonia or an amine is introduced to initiate the reaction. The amine may be gaseous or liquid. Further, the amine may be introduced into the system in a continuous or intermittent manner or at a time (in the case of a liquid amine). During the reaction, the formed water is discharged outside the reaction system together with gaseous substances (excess gaseous amine in the case of the use of hydrogen and a gaseous amine) and passed through the condenser and separator to separate the water from the oleaginous matter. The analysis of the gaseous substances (excess gaseous

EP 0 312 253 B1

amine in the case of the use of hydrogen and a gaseous amine) has revealed that these gaseous substances are substantially free from by–products (e.g., hydrocarbon and amine by–product formed by disproportionation of the starting amine), i.e., has proved that the catalyst of the present invention has high selectivity. Therefore, it was found that these gaseous substances can be reused without any special step of purification. After the completion of the reaction, the reaction mixture itself is subjected to distillation or filtration to separate the reactant from the catalyst. The N–substituted amine obtained by the filtration can be distilled into a very pure form.

Examples

Example 1 and Comparative Examples 1 and 2

A copper/fourth period transition metal element/platinum group element ternary catalyst supported on synthetic zeolite was prepared as follows.

A 1–$\ell$ flask was charged with synthetic zeolite, and an aqueous solution prepared by dissolving copper nitrate, chromium nitrate and palladium chloride in water in a copper : chromium : palladium molar ratio of 4 : 1 : 0.05 was then added thereto, followed by elevation of the temperature while stirring. An aqueous 10 % $Na_2CO_3$ solution was gradually dropwise added thereto at 90°C. The mixture was aged for 1 hr, and the resultant precipitates were collected by filtration, washed with water, dried at 80°C for 10 hr, and baked at 400°C for 3 hr. The amount of the resultant metallic oxide supported on the carrier was 50 %.

Then, a reaction of an alcohol with dimethylamine was conducted in the presence of the above–prepared catalyst. In Comparative Examples, the reaction was conducted in the presence of a copper/nickel/palladium catalyst and a copper/nickel catalyst prepared in the same manner as that described above.

A 1–$\ell$ flask equipped with a condenser and a separator for separating the formed water was charged with 600 g of lauryl alcohol and 1.5 g (0.25 % by weight based on the starting alcohol) of the above–described catalyst. The system was purged with nitrogen while stirring, followed by initiation of the elevation of the temperature. When the temperature reached 100°C, a hydrogen gas was blown into the system through a flowmeter at a flow rate of 10 $\ell$/hr, and the temperature was raised to 200°C. At this temperature, a mixed gas comprising dimethylamine and hydrogen was blown into the reaction system at a flow rate of 40 $\ell$/hr, and the reaction was monitored by measurement of an amine value and gas chromatography.

The results are shown in Table 1.

It was found from the results that as with the Cu/Ni/platinum group element (Pd) ternary catalyst system, the Cu/forth period transition metal element (Cr)/platinum group element (Pd) ternary catalyst system according to the present invention brought about a reduction in the reaction time by half and high conversion of the starting alcohol, i.e., exhibited higher activity than that of the conventional Cu/Ni binary catalyst system (Comparative Example 1), by virtue of the incorporation of a small amount of a platinum group element.

Table 1

| | catalyst composition molar ratio | reaction time (hr) | composition of reaction product (wt%) | | |
|---|---|---|---|---|---|
| | | | unreacted alcohol | lauryl-dimethyl-amine | others |
| Ex. 1 | Cu/Cr/Pd= 4/1/0.05 | 5 | 1.5 | 95.6 | 2.9 |
| Comp. Ex. 1 | Cu/Ni=4/1 | 10 | 5.3 | 89.0 | 5.7 |
| Comp. Ex. 2 | Cu/Ni/Pd= 4/1/0.05 | 5 | 1.8 | 92.3 | 5.9 |

6

Examples 2 to 5 and Comparative Examples 3 and 4

With respect to a catalyst comprising copper, a fourth period transition metal element and a platinum group element, the activity in a reaction of stearyl alcohol with monomethylamine was determined by varying the kind of the fourth period transition metal element among Cr, Mn, Fe, and Zn and using Ru as the platinum group element in the catalyst. These ternary catalysts were prepared in the same manner as that of Example 1.

The results are shown in Table 2.

It was found from the results that when distearylmonomethyl tertiary amine was prepared through a reaction of stearyl alcohol with monomethylamine, the Cu/fourth period transition metal element/Ru catalysts wherein Cr, Mn, Fe or Zn was used as the second component exhibited activity at least twice higher than that of the Cu/Ni catalyst (Comparative Example 3), caused no deterioration of the reaction selectivity and exhibited performance equal or superior to that of the Cu/Ni/Ru catalyst (Comparative Example 4).

## Table 2

|  |  | catalyst* second component metal | reaction time (hr) | composition of reaction product (wt%) | | |
|---|---|---|---|---|---|---|
|  |  |  |  | unreacted alcohol | distearylmono-methylamine | others |
| Ex. | 2 | Cr | 5 | 1.5 | 95.0 | 3.5 |
|  | 3 | Mn | 5 | 1.9 | 93.6 | 4.5 |
|  | 4 | Fe | 5 | 0.5 | 94.5 | 5.0 |
|  | 5 | Zn | 5 | 1.3 | 96.3 | 2.4 |
| Comp. Ex. | 3 | Cu/Ni=4/1 | 10 | 5.6 | 87.8 | 6.6 |
|  | 4 | Ni | 5 | 1.1 | 91.8 | 7.1 |

```
Note:    * Cu/second component/Ru:   molar ratio   3/1/0.03

                                     amount of

                                     support            50 %

         reaction conditions:

             alcohol              :    stearyl alcohol

             amine                :    monomethylamine

             reaction temperature:    200°C

             amount of addition

             of catalyst          :    0.25 % based on alcohol
```

7

Examples 6 to 9 and Comparative Examples 5 and 6

With respect to a catalyst comprising copper, a fourth period transition metal element and a platinum group element, the activity in a reaction of dodecyl alcohol with ammonia was determined by using Zn as the fourth period transition metal element in the catalyst and varying the kind of the platinum group element in the catalyst among Pt, Pd, Ru and Rh. These ternary catalysts were prepared in the same manner as that of Example 1.

The results are shown in Table 3.

It was found from the results that when tridodecylamine was prepared through a reaction of dodecyl alcohol with ammonia, the Cu/Zn/platinum group element catalysts wherein Pt, Pd, Ru or Rh was used as the third component exhibited activity at least twice higher than that of the Cu/Ni catalyst (Comparative Example 5), caused no deterioration of the reaction selectivity and exhibited performance equal or superior to that of the Cu/Ni/Pd catalyst (Comparative Example 6).

Table 3

| | | catalyst* third component metal | reaction time (hr) | composition of reaction product (wt%) | | |
|---|---|---|---|---|---|---|
| | | | | unreached alcohol | tridodecyl-amine | others |
| Ex. | 6 | Pt | 10 | 2.3 | 94.0 | 3.7 |
| | 7 | Pd | 10 | 2.5 | 94.9 | 2.6 |
| | 8 | Ru | 10 | 2.2 | 94.6 | 3.2 |
| | 9 | Rh | 10 | 2.9 | 95.7 | 1.4 |
| Comp. Ex. | 5 | None | 15 | 32.5 | 45.8 | 21.7 |
| | 6 | Pd*[2] | 10 | 3.4 | 91.8 | 4.8 |

Note: *1  Cu/Zn/platinum

group element:        Molar ratio   5/1/0.05

Amount of

support            50 %

*2  Cu/Ni/Pd        :   Molar ratio   5/1/0.05

Amount of

support            50 %

Reaction conditions:

Alcohol            :  Dodecyl alcohol

Amine              :  Ammonia

Amine feed rate    :  10 ℓ/hr

Reaction temperature:  180°C

Amount of addition

of catalyst        :  1.0 % based on alcohol

Example 10 and Comparative Examples 7 and 8

A reaction of lauryl alcohol with ammonia was conducted in the presence of a Cu/Zn/Pd catalyst. In this reaction, ammonia was blown into the system at a feed rate of 30 ℓ/hr. The reaction was monitored by measurement of an amine value and gas chromatography. In Comparative Examples, the same reaction

9

was conducted in the presence of a Cu/Ni/Pd catalyst system and a Cu/Ni catalyst system.

The results are shown in Table 4.

It was found from the results that as with the Cu/Ni/Pd catalyst system (Comparative Example 7), the Cu/Zn/Pd catalyst system enabled a secondary amine to be prepared through a reaction of lauryl alcohol with ammonia with higher selectivity than that in the case of the Cu/Ni catalyst system by varying the ammonia feed rate.

Table 4

| | catalyst* compo- sition | reaction time (hr) | composition of reaction product (wt%) | | |
|---|---|---|---|---|---|
| | | | dilauryl amine | tertiary amine | others |
| Ex. 10 | Cu/Zn/Pd | 5 | 94.5 | 3.6 | 1.9 |
| Comp. Ex. 7 | Cu/Ni/Pd | 5 | 90.8 | 5.0 | 4.2 |
| Comp. Ex. 8 | Cu/Ni | 5 | 58.6 | 27.4 | 14.0 |

Note:  * Cu/Zn/Pd and

Cu/Ni/Pd                :     molar ratio   3/1/0.03

amount of

support                   40 %

Cu/Ni                :     molar ratio   3/1

amount of

support                   40%

reaction conditions:

alcohol                   :   lauryl alcohol

amine                     :   ammonia

amine feed rate       :   30 ℓ/hr

reaction temperature:   180°C

amount of addition

of catalyst               :   0.25 % based on

alcohol

Example 11 and Comparative Exmple 9

A reaction of lauryl alcohol with stearylamine was conducted in the presence of a Cu/Cr/Ru catalyst. In this reaction, stearylamine was introduced at a time in a liquid state into the reaction system. The reaction was monitored by measurement of an amine value and gas chromatography. In Comparative Example, the same reaction was conducted in the presence of a Cu/Ni/Ru catalyst system.

The results are shown in Table 5.

It was found from the results that as with the Cu/Ni/Ru catalyst system (Comparative Example 9), the present catalyst system enabled the preparation of an amine through a reaction of lauryl alcohol with stearyl amine with very high activity and high selectivity.

## Table 5

|  | catalyst* compo- sition | reaction time (hr) | composition of reaction product (wt%) | | |
|---|---|---|---|---|---|
|  |  |  | unreacted alcohol | N-lauryl- stearyl- amine | Others |
| Ex. 11 | Cu/Cr/Ru | 5 | 1.0 | 89.3 | 9.7 |
| Comp. Ex. 9 | Cu/Ni/Ru | 5 | 1.5 | 82.4 | 16.1 |

Note: * Cu/Cr/Ru and

Cu/Ni/Ru             :          molar ratio   4/1/0.1

amount of

support            20 %

reaction conditions:

alcohol              :          lauryl alcohol

amine                :          stearylamine

alcohol/amine = 1

reaction temperature:          180°C

amount of addition

of catalyst            :        1.0 % based on

alcohol

Examples 12 to 15

The effect of the catalyst of the present invention was examined with respect to reactions of various alcohols or aldehydes with dimethylamine to prepare the corresponding tertiary amines. The catalysts were prepared by the impregnation method.

The results are shown in Table 6.

It was found from the above results that the catalyst of the present invention enabled a tertiary amine to be prepared with every high activity and high selectivity even when a branched alcohol, a polyhydric alcohol (glycol), or an aldehyde was used as the starting material and reacted with a secondary amine.

In general, the use of such a branched alcohol, a polyhydric alcohol, or an aldehyde as the starting material brings about an increase in the possibilities of causing side reactions such as decomposition or condensation of these substances. However, it was substantiated that the catalyst having a composition according to the present invention was an excellent catalyst capable of solving these problems.

Table 6

| Ex. | alcohol or aldehyde | catalyst*3 | | reaction temp. (°C) | reaction time (hr) | composition (wt%)*4 | | |
|---|---|---|---|---|---|---|---|---|
| | | composition | amount of addition(%) | | | tertiary amine | unreacted alcohol/ aldehyde | others |
| 12 | oxo alcohol*1 | Cu/Zn/Pd | 0.5 | 200 | 7 | 93.4 | 3.0 | 3.6 |
| 13 | Guerbet*2 alcohol | Cu/Zn/Ru | 0.5 | 230 | 6 | 80.0 | 14.0 | 6.0 |
| 14 | 1,6-hexanediol | Cu/Cr/Pd | 0.5 | 180 | 5 | 91.8 | 3.8 | 4.4 |
| 15 | lauraldehyde | Cu/Cr/Ru | 0.5 | 180 | 5 | 93.5 | 4.0 | 2.5 |

Note: *1 oxo alcohol: a mixture of alcohols having 12 to 13 carbon atoms; a degree of branching of 21% (straight-chain alcohol: 79%)

*2 Guerbet alcohol:

$$\begin{array}{c} R' \\ \diagdown \\ \diagup \\ R'' \end{array} CH\text{--}CH_2OH \quad (\text{total of carbon atoms: 28})$$

*3 Cu/fourth period transition metal element/platinum group element (molar ratio): 5/1/0.02 amount of addition: based on alcohol or aldehyde

*4 The tertiary amine is monoalkyldimethyl tertiary amine (provided that the tertiary amines in the case of 1,6-hexanediol and lauraldehyde are N,N,N'N'-tetramethylhexamethylenediamine and lauryldimethyl tertiary amine, respectively).

Example 16 and Comparative Example 10

A reaction of lauryl alcohol with stearylamine was conducted in the presence of a Cu/Fe/Pd catalyst. In this reaction, stearylamine was introduced at a time in a liquid state into the reaction system. The reaction was monitored by measurement of an amine value and gas chromatography. In Comparative Example, the

13

same reaction was conducted in the presence of a Cu/Ni/Pd catalyst system. The reaction pressure was 50 atm (gauge pressure).

The results are shown in Table 7.

It was found from the results that as with the Cu/Ni/Pd catalyst system (Comparative Example 10), the present catalyst system enabled the preparation of an amine through a reaction of lauryl alcohol with stearylamine with very high activity and high selectivity.

## Table 7

|  | catalyst* compo- sition | reaction time (hr) | composition of reaction product (wt%) | | |
|---|---|---|---|---|---|
|  |  |  | unreacted alcohol | N-lauryl- stearylamine | others |
| Ex. 16 | Cu/Fe/Pd | 5 | 0.2 | 86.3 | 13.5 |
| Comp.Ex.10 | Cu/Ni/Pd | 5 | 0.6 | 83.4 | 16.0 |

Note:    *   Cu/Fe/Pd and

Cu/Ni/Pd            :        molar ratio 6/1/0.01

amount of

support            20 %

reaction conditions:

alcohol            :        lauryl alcohol

amine              :        stearylamine

alcohol/amine = 1

reaction temperature:    160°C

amount of addition

of catalyst        :        1.0 % based on alcohol

Example 17

The reaction mixture prepared in Example 1 was filtered to recover the catalyst therefrom, and the amination reaction was repeatedly conducted under the same conditions.

The results are shown in Table 8.

14

Table 8

| number of runs | reaction time (hr) | composition of reaction product (wt%) | | |
|---|---|---|---|---|
| | | unreacted alcohol | lauryldimethylamine | others |
| 1 | 5 | 1.5 | 95.6 | 2.9 |
| 2 | 5 | 1.8 | 94.5 | 3.7 |
| 3 | 5 | 1.7 | 95.3 | 3.0 |
| 4 | 5 | 1.2 | 96.7 | 2.1 |
| 5 | 5 | 1.7 | 96.5 | 1.8 |

Example 18 and Comparative Example 11

Behenyl alcohol and stearylamine, fed at a molar ratio of 1/1, were reacted with each other in the presence of 2 percent by weight, based on the alcohol, of a catalyst of Cu, Mn and Rh, having a molar ratio of 95/5/0.05 and an amount of support of 40 percent by weight, at 200˚c. The stearylamine was introduced at a time in the form of liquid into the reaction system and the reaction procedure was followed with an amine value and gas chromatographic analysis. The comparative example was carried out in the same way, except for using a catalyst of Cu, Ni and Rh. Results are shown in Table 9. It is found that a corresponding amine can be produced with a high reactivity and a high selectivity from a long chain alcohol and a long chain amine.

Table 9

| | example 18 | comparative example 11 |
|---|---|---|
| catalyst | Cu/Mn/Rh | Cu/Ni/Rh |
| reaction time (hour) | 5 | 5 |
| composition of product mixture (weight percent) | | |
| unreacted alcohol | 2.0 | 3.2 |
| N − stearyl − behenylamine | 88.2 | 80.3 |
| others | 9.8 | 16.5 |

**Claims**

1. A process for preparing an N − substituted amine, which comprises the step of reacting an alcohol or an aldehyde with ammonia, a primary amine or a secondary amine at a temperature from 150 to 250˚C and at a pressure from atmospheric pressure to 101•3 bar (100 atm) gauge, with removal of water formed in the reaction in the presence of a catalyst comprising (1) copper, (2) a metal selected from chromium, manganese, iron and zinc and (3) a metal of the platinum VIII group.

2. A process as claimed in claim 1, in which the molar ratio of the component (1) to (2) ranges from 10/90 to 99/1, the molar ratio of the component (3) to the sum total of (1) and (2) ranges from 0.001 to 0.1.

**Patentansprüche**

1. Verfahren zur Herstellung eines N − substituierten Amins, umfassend den Schritt der Umsetzung eines Alkohols oder eines Aldehyds mit Ammoniak, einem primären Amin oder einem sekundären Amin, bei einer Temperatur von 150 bis 250˚ C bei einem Druck von Atmosphärendruck bis 101,3 bar (100 atm) Manometeranzeige, und Entfernung des in der Reaktion gebildeten Wassers in Gegenwart eines Katalysators, umfassend (1) Kupfer, (2) ein Metall, ausgewählt aus Chrom, Mangan, Eisen und Zink und (3) ein Metall der Platin VIII – Gruppe.

2.  Verfahren nach Anspruch 1, wobei das molare Verhältnis der Komponente (1) zu (2) im Bereich von 10/90 bis 99/1, das molare Verhältnis der Komponente (3) zu der Gesamtsumme von (1) und (2) im Bereich von 0,001 bis 0,1 liegt.

**Revendications**

1.  Procédé pour la préparation d'une amine N – substituée, qui comprend l'étape de réaction d'un alcool ou d'un aldéhyde avec de l'ammoniac, une amine primaire ou une amine secondaire à une température allant de 150 à 250°C et une pression allant de la pression atmosphérique à une pression manomé – trique de 101,3 bar (100 atm), avec élimination de l'eau formée dans la réaction en présence d'un catalyseur comprenant (1) du cuivre, (2) un métal choisi parmi le chrome, le manganèse, le fer et le zinc et (3) un métal du groupe VIII du platine.

2.  Procédé selon la revendication 1, dans lequel le rapport molaire du composant (1) à (2) est compris entre 10/90 et 99/1, le rapport molaire du composant (3) à la somme globale de (1) et (2) est compris entre 0.001 et 0.1.